# EUROPEAN PATENT APPLICATION

(11) **EP 1 544 307 A1**
(43) Date of publication of application: **22.06.2005**
(21) Application number: 03029191.8
(22) Date of filing: 18.12.2003
(51) Int. Cl.: C12Q 1/68, C12N 15/62, C12N 5/10

(54) **LAC9 chimeric receptor and uses thereof**

(71) Applicant: AXXAM S.r.l., 20145 Milano (IT)
(72) Inventor: Lohmer, Stefan, 20132 Milano (IT); Agus, Viviana, 20132 Milano (IT)
(74) Representative: Banfi, Paolo

(57) **Abstract**

The invention discloses chimeric receptors containing the LAC9 transcription factor DNA binding domain, nucleic acid molecules encoding the same and their use in the modulation of the expression of target- or reporter-genes linked to LAC9 response elements in cultured eukaryotic and prokaryotic cells.

SEQUENCE LISTING

<110> AXXAM

<120> LAC9 CHIMERIC RECEPTOR AND USES THEREOF

<130> SCB1242EUR

<160> 3

<170> PatentIn version 3.1

<210> 1
<211> 30
<212> DNA
<213> Kluyveromyces lactis

<400> 1
ctagctagca gccatgggta gtagggcctc 30

<210> 2
<211> 37
<212> DNA
<213> Kluyveromyces lactis

<400> 2
ctagctagcc atggcaaaga aatcaagtga agtaatg 37

<210> 3
<211> 28
<212> DNA
<213> Kluyveromyces lactis

<400> 3
ggaagatctt accggttgca ttggatag 28

## Description

The present invention regards modified intracellular receptors and their use in the modulation of gene expression in eukaryotic and prokaryotic cells. More specifically, the invention provides nuclear receptors carrying the LAC9 DNA binding domain and methods or assays using the same for the study of intracellular receptor activity, receptor-ligand interactions and for the screening of compounds modulating receptor activity.

### BACKGROUND OF THE INVENTION

One of the most interesting field of investigation for both biology and medicine disciplines concerns the molecular mechanism underlying specific gene regulation. In this respect, intracellular nuclear receptors represent the largest known family of eukaryiotic transcription regulators. Their activities are involved in such complex and different physiological events as cell proliferation, differentiation, death, key steps in development, metamorphosis, homeostasis and reproduction.

Nuclear receptors (NR) are phylogenetically related proteins that function as ligand-activated transcription factors representing the key molecules in the signal transduction event from hormone molecule produced for the physiologic process, to the specific gene transcription response. NR superfamily includes both "Nuclear Hormone receptors", for which specific ligands are described, and "Orphan receptors", for which no ligand has been so far identified (1).

All of NRs share common structural features, with a variable N-term region (the A/B domain), a well conserved DNA binding domain (DBD, also called C domain), a not conserved hinge domain (D domain) and a C-term fairly conserved ligand binding domain (LBD, also called E domain) (2,3). Upon ligand stimulation, structural rearrangements occur in ligand-receptor complex, leading NR to bind as homodimer or etherodimer to specific DNA sequences called Hormone Response Elements (HRE). These HRE are Upstream Activating Sequences (UAS) that share a conserved structure constituted by the direct or inverted repetition of the consensus motifs PuGGTCA, separated by variable length nucleotide spacers (4-6).

In contrast to their highly conserved structure, NRs are activated by a wide variety of hydrophobic ligands, comprising both steroid (estrogens, progesterone, glucocorticoid, mineralcorticoid, androgen, etc) and non-steroid lipophilic hormones (VDR, thyroid hormone, retinoic acid, etc...). Unlike hormones for cell surface receptors, lipophilic hormones can pass the plasma membrane and reach the intracellular nuclear receptors, thus initiating the signal transduction mechanisms (1-3).

Due to the widespread relevance of the superfamily of NRs to almost all aspects of human physiology, the role of these receptors in the aetiology of many human diseases is critical and a detailed understanding of these systems has many implications, not only for human biology but also for the understanding and development of new drug treatments.

In the past decades, several methods have been generated for the characterisation of ligand dependent NR activity in living cells (7). Many of them are based on chimeric proteins constituted by the LBD from the NR of interest fused with an heterologous DBD (usually from the *Saccharomyces cerevisiae* GAL4 transcription factor). The chimeric transcription factor activity is modulated by the receptor ligand and induces the transcription of specific target genes cloned downstream of the specific UAS recognised by GAL4-DBD (8,9).

Due to the low cross-reactivity of such a chimeric protein with the host mammalian genes, such tools are not only suitable for the development of ligand-regulated transcription assay for the discovery of new NR-ligand molecule for NRs, but also in the field of inducible gene expression systems, protein-protein interaction assays and for important "in vivo" gene therapy applications (10-14).

### DESCRIPTION OF THE INVENTION

The present invention provides a chimeric receptor comprising the *Kluyveromyces lactis* LAC9 transcription factor DNA binding domain (DBD) fused to a nuclear receptor or a portion thereof containing the ligand binding domain (LBD). The chimeric receptor forms functional homodimers upon ligand binding and specifically activates transcription through LAC9 response elements. Cells expressing the chimeric receptor and bearing appropriate response elements functionally linked to a target or reporter gene provide a valuable tool for the study of nuclear receptor function or regulation and ligand-receptor or protein-protein interactions.

According to a first embodiment, the invention provides a method for modulating the expression of a target gene in a cultured eukaryotic or prokaryotic cell, which comprises:
A) introducing into the cell:
   i) a vector encoding the *Kluyveromyces lactis* LAC9 transcription factor DNA binding domain (DBD) fused to a nuclear receptor or a portion thereof containing the ligand binding domain,
      and, simultaneously or separately,
   ii) a vector containing a LAC9 response element operatively linked to the target gene;
B) contacting the cell with a ligand of the nuclear receptor.

In a further embodiment, the invention is directed to a method for the identification of ligands of a nuclear receptor in a cultured eukaryotic or prokaryotic cell, which comprises:
A) introducing into the cell:
   i) a vector encoding the *Kluyveromyces lactis* LAC9 transcription factor DNA binding domain (DBD) fused to a nuclear receptor or a portion thereof containing the ligand binding domain,
      and, simultaneously or separately,
   ii) a vector containing a LAC9 response element operatively linked to a reporter gene;
B) contacting the cell with a candidate ligand of said receptor.
C) determining the activity (expression) of the reporter gene.

In a yet further embodiment, the invention provides a method for identifying the functional domains involved in the activation of a nuclear receptor, which comprises:
A) introducing into a cultured eukaryotic or prokaryotic cell:
   i) a vector encoding the *Kluyveromyces lactis* LAC9 transcription factor DNA binding domain (DBD) fused to different portions of the intracellular receptor,
      and, separately or simultaneously,
   ii) a vector containing a LAC9 response element operatively linked to a reporter gene;
B) contacting the cell with a receptor ligand;
C) determining the activity (expression) of the reporter gene.

Another embodiment of the invention regards a method for characterizing the activity of a mutant variant of intracellular receptor, which comprises:
A) introducing into a cultured eukaryotic or prokaryotic cell:
   i) a vector encoding the *Kluyveromyces lactis* LAC9 transcription factor DNA binding domain (DBD) fused to the mutant intracellular receptor or a portion thereof containing the ligand binding domain,
      and, separately or simultaneously,
   ii) a vector containing a LAC9 response element operatively linked to a reporter gene;
B) contacting the cell with a receptor ligand;
C) determining the activity (expression) of the reporter gene.

The invention additionally provides nucleic acid molecules encoding the *Kluyveromyces lactis* LAC9 transcription factor DNA binding domain (DBD) fused to a nuclear receptor, preferably a steroid/thyroid receptor, or a portion thereof containing the ligand binding domain, as well as vectors and eukaryotic or prokaryotic host cells carrying such nucleic acid molecules. The LAC9 DBD can also be used to generate chimeric proteins for the so called "two-hybrid" protein-protein interaction screening assays.

### DETAILED DESCRIPTION OF THE INVENTION

According to the present invention, a chimeric receptor containing a *Kluyveromyces lactis* LAC9 transcription factor DNA binding domain (DBD) fused to a nuclear receptor or a portion thereof containing the ligand binding domain, is used for the modulation of the expression of a target or a reporter gene operatively linked to LAC9 response elements in a cultured eukaryotic or prokaryotic cell. The chimeric receptor combined with a suitable reporter system is particularly useful for the study of receptor function, ligand-receptor interactions, and for the screening of compounds modulating receptor activity.

Any nuclear receptor that undergoes homodimerization upon ligand binding, especially the members of the steroid/thyroid hormone receptor family, can be used in accordance with the present invention.

The LAC9-DBD can be introduced in different positions of the receptor protein, preferably at the N- or C-terminus, provided that ligand binding and receptor homodimerization are not negatively affected. In addition, the modified receptor may contain one or more exogenous transactivation domains, such as tau1, tau2 or VP 16 transactivation domains.

The DNA binding domain (DBD) of LAC9 protein is determined on the basis of known functional and structural similarities between this protein and the *Saccharomyces cerevisiae* GAL4 transcription factor (15-18). Reference LAC9 nucleotide and amino acid sequences are reported in GenBank Acc. No. M15210 and AAA35266, respectively. The LAC9-DBD can be modified, e.g. by deletion or addition of one or more amino acid residues, provided that the transcriptional activity profile of the chimeric receptor, in terms of ligand responsiveness and reporter gene expression, is maintained or increased.

Preferably, the LAC9-DBD spans residues 85-178 of the reference LAC9 protein, more preferably residues 85-228.

The chimeric receptor can be prepared using conventional genetic engineering techniques. For example, the cDNAs coding for LAC9 and for the nuclear receptor are amplified by PCR or constructed "in vitro" with synthetic oligonucleotides spanning the desired nucleotide region, and the products are recombined making use of suitable restriction sites, naturally occurring or artificially introduced into the oligonucleotides used for amplification or for *in vitro* construction. The expression vector may contain, besides the recombinant construct, a promoter, a ribosome binding site, an initiation codon, a stop codon or a consensus site for transcription enhancers. The vector can also comprise a selection marker for isolating the host cells containing the DNA construct.

The activity and functionality of the chimeric receptor can be detected by means of a reporter system containing LAC9 response elements operatively linked to a reporter gene, whereby an increased signal associated with the activity of the reporter gene indicates ligand-mediated activation of the receptor.

LAC9 response elements can be amplified by PCR from *K. lactis* GAL1-GAL10 intergenic region or constructed "in vitro" with synthetic oligonucleotides. Typically, the response elements will contain the 17-mer consensus sequence 5'-CGGNNNNN(A/T)NNNNNCCG-3' (where "N" represents any nucleotide) (19-22), for example the four motifs present in the *K. lactis* GAL1-GAL10 intergenic region. The reporter vector may contain multiple consensus sequence repeats, in various orientation/location, and different nucleotide residues with respect to LAC9 UAS (Upstream Activated Sequences).

The response elements are operatively linked to a reporter- or target-gene coding sequence, the expression of which is inducible by binding of the ligand-activated receptor to LAC9 response elements. Examples of reporter genes include the luciferase, green fluorescent protein, chloramphenicol acyl-transferase genes. The reporter vector may also comprise a selection marker for isolating the host cells containing the DNA construct.

According to the invention, the expression and reporter vectors may be simultaneously or separately transfected into a suitable host cell. In a typical application, transfected cells are incubated with a test compound and receptor activation is determined by reporter gene expression. Since only the modified receptor is able to transactivate reporter gene expression upon ligand binding, the use of receptor-negative cells is not needed.

Any prokaryotic or eukaryotic cells, such as bacteria, yeast or mammalian cells can be used as cellular hosts. Mammalian cell lines, such as CHO K1, HeLa, HepG2 and Hek-293 cells, are preferred. Alternatively, non-mammalian cells, such as bacteria or fungi, can be employed. Once the host cell type has been selected, the genetic constructs is introduced by calcium phosphate precipitation, electroporation or other conventional transfection methods. Cells are grown in appropriate media and assayed for receptor activity, for example by adding the candidate compound to the culture medium and testing reporter gene activity.

The ability of LAC9 chimeric nuclear receptors to transactivate reporter gene expression was assayed in CHO K1 mammalian cells transfected with an expression vector encoding the Estrogen Receptor alpha (GeneBank Acc. No. NM_000125) fused to the LAC9-DBD and with a vector bearing LAC9 response elements functionally linked to the firefly Luciferase reporter gene.

The human Estrogen receptor alpha Ligand Binding Domain (aa 282 - 595; GenBank Acc. No. NP_000116) (23) was isolated by PCR amplification and cloned in frame with LAC9-DBD (aa 85-228) into an expression vector, under the control of CMV immediate early promoter. The expression vector containing the chimeric protein also included a fluorescent marker gene as internal control for transfection efficiency. The reporter vector was obtained by inserting *K. lactis* GAL1-GAL10 intergenic region (GenBank Acc. No. X07039; bp706-983) upstream of the firefly Luciferase reporter gene. After co-transfection, the cells were added with the estrogen receptor ligand β-estradiol and reporter gene expression was monitored at different incubation times using a conventional luminometer. Luciferase light emission was detected from detergent-lysed cells by contact with a Luciferin solution. To correlate reporter gene activation with chimeric receptor activity, the transfection efficiency was normalized by fluorescent marker gene expression.

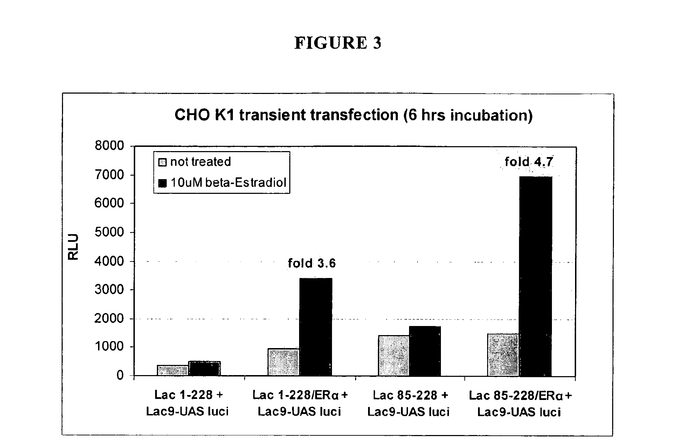

In these experiments, the chimeric receptor was able to specifically activate luciferase gene expression in the presence of β-estradiol agonist, with a 4 to 5 fold luciferase induction over non-transfected cells. In the absence of specific ligand, no reporter gene expression was observed. The LAC9-DBD construct alone was not able to activate reporter gene expression upon ligand addiction (Fig. 3).

Further experiments demonstrated the higher efficacy of ligand-regulated reporter gene transactivation by LAC9-chimeric receptor as compared with conventional methods utilising the *S*. *cerevisiae* GAL4 DBD fused to a NHR LBD in combination with a reporter gene operatively linked to GAL4 specific UAS.

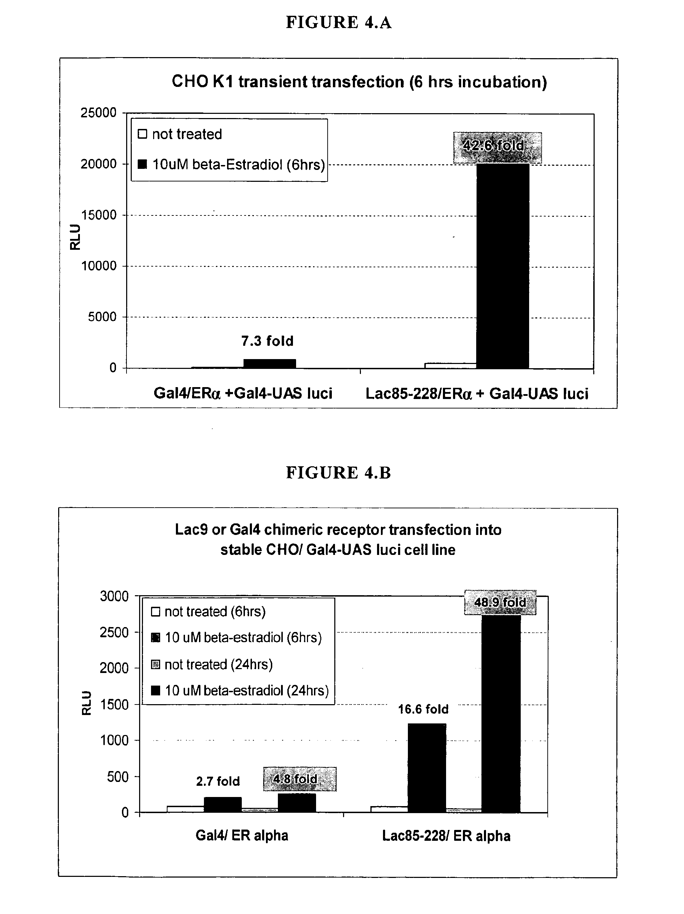

In transfection experiments of CHO K1 cells with Gal4 UAS-Luciferase reporter vector and with the Estrogen receptor-alpha LBD cloned in either Gal4 or LAC9-DBD containing vector, the LAC9-ERα chimera resulted 6 to 10 fold more active on Gal4 UAS reporter vector than the conventional Gal4 chimeric receptor, as shown by β-estradiol induced luciferase activity (Fig. 4).

The results of these experiments demonstrate: i) the ability of LAC9 chimeric receptors to specifically activate gene expression by binding LAC9 Response Elements and, ii) an improved ligand-regulated transcription activity of LAC9 chimeric receptors in comparison to GAL4 chimeric receptors.

Cells expressing both a LAC9-chimeric receptor and a suitable LAC9-UAS reporter vector can be used to test candidate molecules for their effects on the modulation of nuclear receptors.

In addition, the LAC9 DBD can be used to generate chimeric proteins for two-hybrid protein-protein interaction screening assays, where two different proteins are respectively fused to LAC9 DBD and to an heterologous transcriptional activating domain, such as the Herpes simplex virus type 1 VP 16 protein.

High throughput screening assays can be designed, utilising LAC9-UAS/reporter gene as the readout system for LAC9 chimera receptor activity. The sensitivity of the system as well as its high signal to noise ratio allow using small assay-volumes.

### DESCRIPTION OF THE DRAWINGS

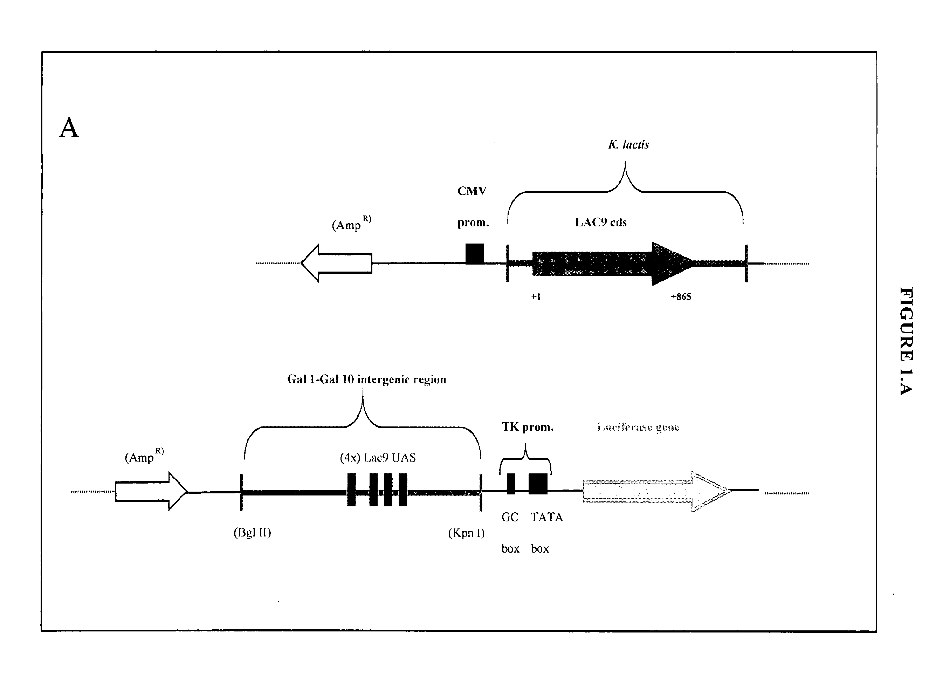

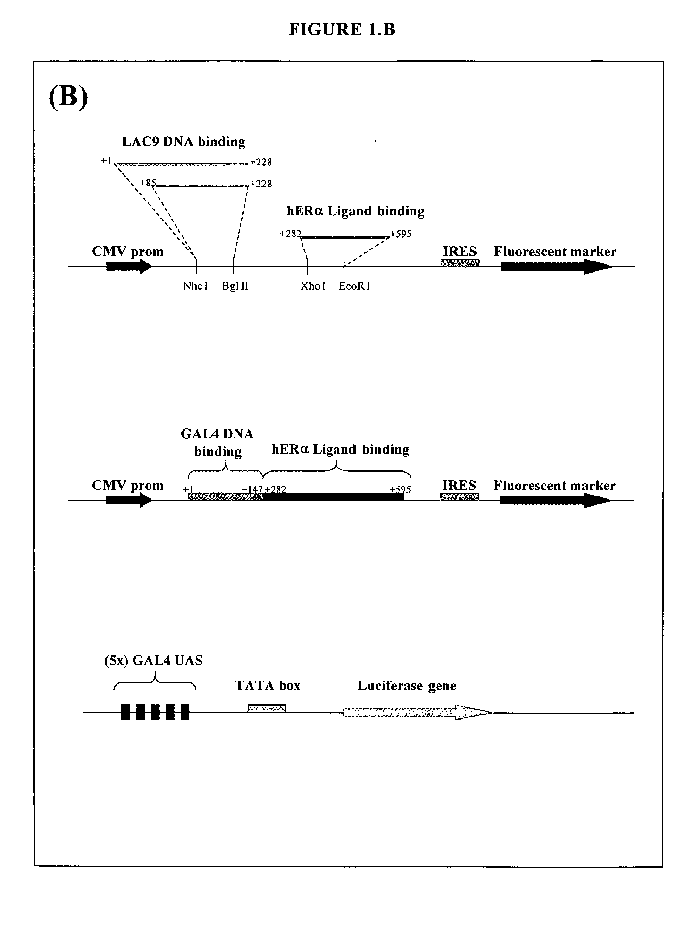

**Fig. 1.** LAC9 full-length receptor and *K. lactis* UAS reporter vector (Fig. 1A): the full length LAC9 cds was cloned under the control of CMV promoter. The intergenic region of *K*. *lactis* GAL1-GAL10 genes (containing four LAC9 response elements) has been cloned as a BgIII/ KpnI fragment (278 bp) upstream of a minimal TK promoter, into a Luciferase reporter vector. Chimeric receptor constructs (Fig. 1B): two different LAC9-DBD constructs were generated comprising the aminoacid 1-228 or 85-228 of LAC9; GAL4-DBD (containing aa residues 1-147) was cloned from a commercial vector; all the DBD were cloned into a bicistronic vector expressing a fluorescent protein marker through an IRES element. Ligand binding domain of Estrogen receptor-alpha (aa 282-595) was cloned in frame into both the LAC9 and GAL4-DBD vectors. A conventional GAL4-UAS reporter vector, containing 5 repetition of the 17bp UAS element of GAL4 was also used.

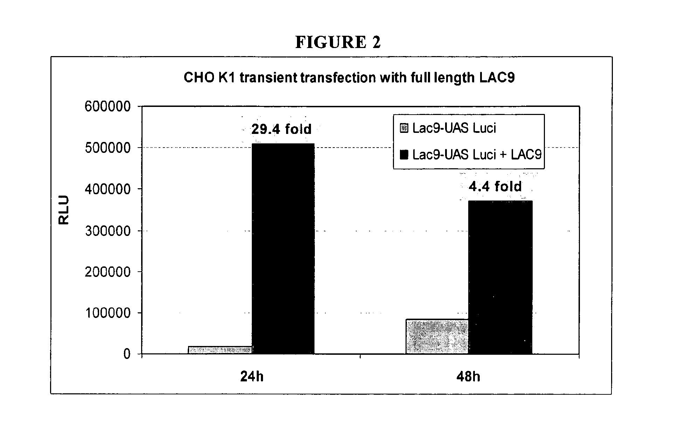

**Fig. 2.** Constitutive activity of full-length LAC9 in CHO-K1 cell line. 10,000 cells/well were transfected in 96 well/plates, 24 hrs after seeding, with 30 ng DNA/well for each plasmid (total DNA amount was maintained including 30 ng DNA/well of pBluescript in LAC9-UAS Luci transfection). Luciferase production was tested 24 or 48 hrs after transfection with 3 sec luminescence reading. Fold indicates the ratio between RLU of LAC9+UAS transfection and basal level (UAS alone).
**Fig. 3**. LAC9 chimeric receptor activity. The two different LAC9-DBD constructs (Lac 1-228 and Lac 85-228) and the correspondent chimeric receptors containing also the ERα LBD (Lac1-228/ERα and Lac85-228/ERα) were co-transfected with the LAC9-UAS reporter vector. After a period of 24 hrs, cells were incubated for 6 hrs with 10 µM b-estradiol, then Luciferase production was measured.
**Fig. 4.** Comparison of LAC9 and GAL4 chimeric receptor efficiency. GAL4/ERα or LAC85-228/ERα chimeras were transfected together with GAL4-UAS reporter vector (fig. 4A: co-transient experiment; fig. 4B: transient transfection of a stable GAL4-UASLuci cell line). 24 hrs later, cells were incubated for 6 hrs (fig. 4A and 4B) or 24 hrs (fig. 4B) with 10 µM β-estradiol, then the luciferase production was tested.

### EXAMPLES

### 1. LAC9 transcriptional activity in CHO-K1 mammalian cell line system

To verify whether the *Kluyveromyces lactis* LAC9 transcription factor was able to efficiently transactivate the expression of reporter gene through the LAC9-UAS in hamster CHO-K1 cell line (as previously reported for human HepG2 cell line) (24), the full-length LAC9 cDNA was initially transfected together with a luciferase reporter vector containing the LAC9 specific UASs, into the CHO-K 1 cell line (maps of the transfected vectors are shown in fig. 1A). 24 hrs before experiments, 10,000 c/w were plated into 96 well plates, then 60 ng of total DNA were transiently transfected by lipocationic transfection reagent, following the supplier instruction. Luciferase production was tested 24 and 48 hrs after transfection by lysis of the cells with Triton detergent and injection of Luciferine solution. Light emission was measured for 3 second with Berthold Luminometer instrument. Relative Light Unit are reported in Table 1 (value indicated are mean RLU from 8 wells):

**Table 1**

| **Luciferase production upon full-length LAC9 and LAC9-UAS Luci reporter vector transfection** | | |
|---|---|---|
| **Vector** | **Luminescence (RLU)** | |
| | **24 hrs** | **48 hrs** |
| **Lac9-UAS Luci** | **17372** | **83809** |
| **Lac9-UAS Luci + LAC9 f.I.** | **510104** | **372140** |
| **fold induction** | **29.4** | **4.4** |

As shown in Fig. 2, expression of LAC9 transcription factor into CHO-K1 cell line is able to efficiently induce luciferase expression from the reporter plasmid containing the 4 LAC9-UAS, with a fold induction of about 30.

### 2. LAC9-ERalpha chimeric receptor construction and ligand regulated transactivation of LAC9-UAS

LAC9-Estrogen Receptor alpha chimeric receptors were obtained by "in frame" cloning of LAC9-DBD residues 1 to 228 ("Lac1" construct) or 85 to 228 ("Lac85" construct) with ERα LBD residues (aa 282-595); primer sets utilised for PCR amplification of LAC9-DBD are described in Materials and Methods. For the "Lac85" construct, an artificial Kozak consensus sequence and an ATG were introduced in the 5' primer sequence, in order to promote the eukaryotic transcription and translation of the construct. The two LAC9-DBD constructs ("Lac1" and "Lac85") were cloned into a mammalian bi-cistronic expression vector, containing at the 3' end an IRES element driving the translation of a fluorescent protein marker (see fig. 1B). Corresponding chimeric receptor were obtained by cloning the ERα LBD (aa 282-595) into the LAC9-DBD constructs, thus generating the Lac1- ERα and Lac85- ERα chimeric receptor vector (see fig. 1B). Chimeric receptors activity was tested by co-trasfection of CHO-K1 mammalian cell line with the LAC9-UAS luciferase reporter vector described into example 1, together with one of the said chimeric constructs ("Lac1-ERα" or "Lac85-ERα") or the corresponding DBD vectors ("Lac1" or "Lac85"). 10,000 cells/well were plated in 96 well/plates and after 24 hrs, transient experiments were performed by transfecting 60 ng total DNA/well with lipocationic transfection reagent, following the supplier indications. After 24 hrs, ligand regulated activity of the constructs were tested by incubating the cells in serum-free medium containing 10 µM β-estradiol for 6 hrs, at 37°C, 5% CO₂. Luciferase production was detected by Triton lysis of the cells followed by injection of luciferine solution. Light emission was measured for 3 second with Berthold Luminometer instrument. Relative Light Unit are shown in Table 2 (value indicated are mean RLU from 8 wells):

**Table 2**

| **Luciferase production upon transfection ofLAC9 chimeric receptors and LAC9 UAS luci vector** | | | | |
|---|---|---|---|---|
| | | **Luminescence (RLU)** | | |
| **Incubation (6 hrs)** | **Vector (60 ng total DNA/well)** | | | |
| | **LAC9 UAS luci+ Lac 1-228** | **LAC9 UAS luci+ Lac 1-228/ERα** | **LAC9 UAS luci+ Lac 85-228** | **Lac9 UAS luci+ Lac 85-228/ERα** |
| **not treated** | **350** | **939** | **1405** | **1478** |
| 10 µM **beta-Estradiol** | **513** | **3418** | **1748** | **7007** |
| **fold** | **1.5** | **3.6** | **1.2** | **4.7** |

As shown in Fig. 3, both the chimeric constructs were able to transactivate Luciferase expression only in the presence of the specific ligand β-estradiol. In particular the "Lac85-ERα" chimera shown the best fold induction in terms of luciferase expression over the basal level. No ligand-dependent transcriptional activity was detected for the corresponding LAC9-DBD alone constructs.

### 3. LAC9 chimeric receptor efficiency on homologue reporter system

Ligand-regulated activity of the LAC9 chimeric receptor was tested in comparison to the existing Gal4 chimeric receptor system, in order to verify the efficiency of the chimeric receptor of present invention in comparison to existing homologous chimeric construct.

Ga14-ERα chimeric construct was obtained by "in frame" cloning of the GAL4 DNA binding domain (residues 1-147) followed by ERα LBD residues (aa 282-595) (see fig. 1B); analogue LAC9 chimeric receptor containing the LAC9-DBD (residues 85 to 228) fused to the same ERα LBD residues (see example 2) was utilised for the experiments; both the chimeric receptors were cloned into the bi-cistronic expression vector described in example 2.

As reporter vector for both the chimeric constructs, a plasmid containing the luciferase gene under the control of 5 repetitions of GAL4 UAS was used (see fig. 1B), as it's known that LAC9 UASs conform to the same consensus sequence as GAL4 recognised ones:

5'-CGGNNNNN(A/T)NNNNNCCG-3'.

The activity of the chimeric receptors was evaluated both in transient co-transfection experiments (by using the reporter plasmid in combination with each of the chimeric constructs) and "semi-transient" experiments (by transfecting chimeric receptors into a cell line stably expressing the reporter vector). 24 hours before experiment, 10,000 c/w of CHO-K1 mammalian cell line (wt cell line, or stably transfected cells) were plated in 96 well/plates; then 60 ng total DNA/well was transfected with lipocationic transfection reagent, following the supplier indications. After 24 hours, ligand regulated activity of the receptors was evaluated by incubating the cells for 6 hrs (wt and stable GAL4-UAS cell line) or 24 hours (only GAL4-UAS cell line) into serum-free medium containing 10 µM of the specific ligand β-estradiol. After cell lysis by Triton detergent, Luciferase production was detected by injection of luciferine solution and 3 second light emission measurement with Berthold Luminometer instrument. Relative Light Unit are shown in Table 3 (values indicated are mean RLU from 8 wells):

**Table 3**

| **Luciferase production upon co-transfection of Lac9/ or Ga14/ERα chimeric receptor and Ga14 UAS luci vector** | | | | |
|---|---|---|---|---|
| | **Luminescence (RLU)** | | | |
| | **Vector (60ng total DNA/well)** | | | |
| | **Ga14-UAS luci +** | **Ga14-UAS luci +** | **(Gxa14-UAS stable cell line)** | |
| **Incubation (6 hrs)** | **Ga14/Era** | **Lac 85-228/Erα** | **Ga14/Erα** | **Lac 85-228/Erα** |
| **not treated** | **125** | **472** | **76** | **74** |
| 10uM beta-Estradiol | 910 | 20124 | 206 | 1226 |
| fold | 7.3 | 42.6 | 2.7 | 16.6 |
| | | | | |

| | **Ga14-UAS luci +** | **Ga14-UAS luci +** | **(Ga14-UAS stable cell line)** | |
|---|---|---|---|---|
| **Incubation (24 hrs)** | **GaI4/Erα** | **Lac 85-228/Erα** | **Gal4/Erα** | **Lac 85-228/Erα** |
| **not treated** | **/** | **/** | **54** | **56** |
| **10uM beta-Estradiol** | **/** | **/** | **258** | **2738** |
| **fold** | **/** | **/** | **4.8** | **48.9** |

As shown in Fig. 4, LAC9 chimeric receptor shown 6 to 10 fold higher activity in ligand regulated transactivation of luciferase reporter gene than the conventional Gal4 chimeric construct, both in transient and in "semi-transient" experiments.

### MATERIALS AND METHODS

### Reagents

Restriction enzymes were purchased from New England Biolabs and used according to supplier's instructions. Platinum Pfx DNA polymerase, reagents for PCR, chemically competent cells of E. *coli* strains TOP10 F' and all cell culture reagents, were from GIBCO (USA). Oligonucleotides were purchased from Primm (Milan). Rapid DNA ligation kit and Fugene transfection reagent were purchased from Roche (Basel, CH). Luciferase was from P.J.K (Kleinblittersdorf, D). Beta-estradiol was from Sigma (St. Louis, MO). All other chemicals were from standard sources and were of reagent grade or better.

### Reporter vector construction

The LAC9-reporter vector was created by the cloning of *K. lactis* GAL1- GAL10 intergenic region (278 bp, containing the 4 UAS elements recognised by LAC9 transcription factor) into a luciferase reporter vector (see fig. 1A)

The GAL4-reporter vector utilised for comparative studies was purchased from Stratagene (La Jolla, CA), and contains 5 repetitions of the 17 nt GAL4 binding element in front of the Luciferase reporter gene (see fig. 1B).

### Chimeric receptor construction

The chimeric receptor was obtained by fusing the Ligand binding domain of the Estrogen receptor-alpha (ERα) to the DNA binding domain of either GAL4 or LAC9 transcription factor.

To obtain the LAC9 DNA binding domain, two primer sets were designed; the first one (LAC9 fwd/ LAC9-228rev) allows the amplification of the aminoacid residues 1 to 228 of LAC9 protein (GenBank Acc. No. AAA35266); the second one (LAC9-85fwd/ LAC9-228rev) amplifies the region corresponding to aminoacid residues 85 to 228; in this case a Kozak consensus sequence and an artificial ATG codon were introduced in the 5' of forward primer.

The oligonucleotide primers utilised were the following:
LAC9 fwd: 5'-CTAGCTAGCAGCCATGGGTAGTAGGGCCTC-3'
LAC9-85fwd: 5'-CTAGCTAGCCATGGCAAAGAAATCAAGTGAAGTAATG-3'
LAC9-228rev:

5'-GGAAGATCTTACCGGTTGCATTGGATAG-3'

Each of the two amplified fragments was cloned into a bi-cistronic mammalian expression vector (purchased from Clontech) that allows the simultaneous transcription of a fluorescent protein marker, through an Internal Ribosome Entry Site (IRES) element (see fig. 1B), thus generating the so-called LAC 1-228 or LAC 85-228 vector.

The analogue GAL4 DNA binding domain containing vector was constructed by cloning the aminoacid residues 1-147 of GAL4 protein into the same bi-cistronic vector.

To obtain chimeric receptors, the ER-α Ligand binding domain (aminoacid residues 282 to 595; Acc.No. NP_000116) was PCR amplified from breast cDNA and "in-frame" cloned as an XhoI/EcoRI fragment into the LAC9 or GAL4 DNA binding containing vector (see fig. 1B).

All the chimeric constructs obtained were verified by full-length dideoxy sequencing.

### CHO cell culture and transfection

CHO-K1 cells were cultured under standard humidified conditions at 37°C and 5% CO₂. Cells were maintained in DMEM/F12 + FBS 10% + Pen/Strep 1 % + Pyruvate 1.6 mM + NaHCO₃ 0.2%, all reagents were from GIBCO-Life Technologies. For the transient transfection, 10,000 cells/well were plated into 96 well/plates; then, after 24 hours, 60 ng total DNA was added to the cells together with lipocationic transfection reagent, according to supplier's instructions.

### Luciferase reporter assay

Luciferase production was tested 24 or 48 hours after transfection; after cell lysis with Triton detergent, Luciferine solution was injected and 3 sec luminescence measurement was performed with standard Berthold Luminometre. For the chimeric receptor stimulation, 6 hrs or 24 hrs incubation with 10 µM beta-estradiol (Sigma) was performed in Optimem medium (GIBCO), before luciferase detection.

### REFERENCES

1. Olefsky JM. (2001) Nuclear receptor minireview series. J Biol Chem. Oct 5; 276(40):36863-4.
2. Robinson-Rechavi M, Escriva Garcia H, Laudet V (2003). The nuclear receptor superfamily. J Cell Sci. Feb 15;116(Pt 4):585-6.
3. Aranda A, Pascual A. (2001) Nuclear hormone receptors and gene expression. Physiol Rev. Jul;81(3):1269-304.
4. Kato S, Sasaki H, Suzawa M, Masushige S, Tora L, Chambon P, Gronemeyer H. (1995) Widely spaced, directly repeated PuGGTCA elements act as promiscuous enhancers for different classes of nuclear receptors. Mol Cell Biol. Nov;15(11):5858-67.
5. Hart SM. (2002) Modulation of nuclear receptor dependent transcription. Biol Res.;35(2):295-303.
6. Guarente L. (1988) UASs and enhancers: common mechanism of transcriptional activation in yeast and mammals. Cell. Feb 12;52(3):303-5.
7. Shiau AK, Coward P, Schwarz M, Lehmann JM. (2001). Orphan nuclear receptors: from new ligand discovery technologies to novel signaling pathways. Curr Opin Drug Discov Devel. Sep;4(5):575-90.
8. Jausons-Loffreda N, Balaguer P, Roux S, Fuentes M, Pons M, Nicolas JC, Gelmini S, Pazzagli M. (1994) Chimeric receptors as a tool for luminescent measurement of biological activities of steroid hormones. J Biolumin Chemilumin. May-Jun;9(3):217-21.
9. Rudakoff B, Undisz K, Mayer G, Sobek L, Kaufmann G, Thiericke R, Grabley S, Munder T. (1999) Dual reporter systems in yeast and mammalian cells for assessing progesterone receptor modulators. J Cell Biochem. Apr 1;73(1):126-36.
10. Lewandoski M. (2001) Conditional control of gene expression in the mouse. Nat Rev Genet. Oct;2(10):743-55.
11. White MA. (1996) The yeast two-hybrid system: forward and reverse. Proc Natl Acad Sci U S A. Sep 17;93(19):10001-3.
12. Zhao B, Magdaleno S, Chua S, Wang YL, Burcin M, Elberg D, Finegold M, Tsai S, DeMayo FJ. (2000) Transgenic mouse models for lung cancer. Exp Lung Res. Dec;26(8):567-79.
13. Wang Y, O'Malley BW Jr, Tsai SY, O'Malley BW (1994). A regulatory system for use in gene transfer. Proc Natl Acad Sci USA. Aug 16;91(17):8180-4.
14. Louvion JF, Havaux-Copf B, Picard D (1993). Fusion of GAL4-VP16 to a steroid-binding domain provides a tool for gratuitous induction of galactose-responsive genes in yeast. GeneSep 6;131 (1):129-34.
15. Gardner KH, Anderson SF, Coleman JE (1995). Solution structure of the *Kluyveromyces lactis* LAC9 Cd2 Cys6 DNA-binding domain. Nat Struct Biol. Oct;2(10):898-905.
16. Breunig KD, Kuger P (1987). Functional homology between the yeast regulatory proteins GAL4 and LAC9: LAC9-mediated transcriptional activation in *Kluyveromyces lactis* involves protein binding to a regulatory sequence homologous to the GAL4 protein-binding site. Mol Cell Biol. Dec;7(12):4400-6.
17. Wray LV Jr, Witte MM, Dickson RC, Riley MI (1987). Characterization of a positive regulatory gene, LAC9, that controls induction of the lactose-galactose regulon of *Kluyveromyces lactis:* structural and functional relationships to GAL4 of Saccharomyces cerevisiae. Mol Cell Biol. Mar;7(3):1111-21.
18. Salmeron JM Jr, Johnston SA (1986). Analysis of the *Kluyveromyces lactis* positive regulatory gene LAC9 reveals functional homology to, but sequence divergence from, the Saccharomyces cerevisiae GAL4 gene. Nucleic Acids Res. Oct 10;14(19):7767-81.
19. Godecke A, Zachariae W, Arvanitidis A, Breunig KD (1991). Coregulation of the *Kluyveromyces lactis* lactose permease and beta-galactosidase genes is achieved by interaction of multiple LAC9 binding sites in a 2.6 kbp divergent promoter. Nucleic Acids Res. Oct 11;19(19):5351-8.
20. Halvorsen YD, Nandabalan K, Dickson RC (1991). Identification of base and backbone contacts used for DNA sequence recognition and high-affinity binding by LAC9, a transcription activator containing a C6 zinc finger. Mol Cell Biol. Apr;11 (4):1777-84.
21. Leonardo JM, Bhairi SM, Dickson RC (1987). Identification of upstream activator sequences that regulate induction of the beta-galactosidase gene in *Kluyveromyces lactis.* Mol Cell Biol. Dec;7(12):4369-76.
22. Ruzzi M, Breunig KD, Ficca AG, Hollenberg CP (1987). Positive regulation of the beta-galactosidase gene from *Kluyveromyces lactis* is mediated by an upstream activation site that shows homology to the GAL upstream activation site of Saccharomyces cerevisiae. Mol Cell Biol. Mar;7(3):991-7.
23. Webster NJ, Green S, Tasset D, Ponglikitmongkol M, Chambon P (1989). The transcriptional activation function located in the hormone-binding domain of the human oestrogen receptor is not encoded in a single exon. EMBO J. May;8(5):1441-6.
24. Schulz WA, Ebling B, Hasse A, Zenke F, Breunig K (1993). Highly efficient transactivation by the yeast *Kluyveromyces lactis* transcription factor LAC9 and its inhibition by the negative regulator GAL80 in mammalian cells. Biol Chem Hoppe Seyler. May;374 (5):313-8.

## Claims

1. A method for modulating the expression of a target gene in a cultured eukaryotic or prokaryotic cell, which comprises:
A) introducing into the cell:
i) a vector encoding the *Kluyveromyces lactis* LAC9 transcription factor DNA binding domain (DBD) fused to a nuclear receptor or a portion thereof containing the ligand binding domain,
and, simultaneously or separately,
ii) a vector containing a LAC9 response element operatively linked to the target gene;
B) contacting the cell with a ligand of the nuclear receptor.

2. A method for the identification of ligands of a nuclear receptor in a cultured eukaryotic or prokaryotic cell, which comprises:
A) introducing into the cell:
i) a vector encoding the *Kluyveromyces lactis* LAC9 transcription factor DNA binding domain (DBD) fused to a nuclear receptor or a portion thereof containing the ligand binding domain,
and, simultaneously or separately,
ii) a vector containing a LAC9 response element operatively linked to a reporter gene;
B) contacting the cell with a candidate ligand of said receptor.
C) determining the activity of the reporter gene.

3. A method for identifying the functional domains involved in the activation of a nuclear receptor, which comprises:
A) introducing into a cultured eukaryotic or prokaryotic cell:
i) a vector encoding the *Kluyveromyces lactis* LAC9 transcription factor DNA binding domain (DBD) fused to different portions of the nuclear receptor,
and, separately or simultaneously,
ii) a vector containing a LAC9 response element operatively linked to a reporter gene;
B) contacting the cell with a receptor ligand;
C) determining the activity of the reporter gene.

4. A method for characterizing the activity of a mutant variant of a nuclear receptor, which comprises:
A) introducing into a cultured eukaryotic or prokaryotic cell:
i) a vector encoding the *Kluyveromyces lactis* LAC9 transcription factor DNA binding domain (DBD) fused to the mutant nuclear receptor or a portion thereof containing the ligand binding domain,
and, separately or simultaneously,
ii) a vector containing a LAC9 response element operatively linked to a reporter gene;
B) contacting the cell with a receptor ligand;
C) determining the activity of the reporter gene.

5. A method according to claims 1-4, wherein the nuclear receptor is a member of the steroid/thyroid hormone receptor family.

6. A method according to claim 1, wherein the expression of the target gene is modulated by the nuclear receptor ligand to obtain an inducible expression system.

7. A method according to claim 2, wherein said ligand is an agonist or antagonist of the nuclear receptor.

8. A method according to claims 1-4, wherein the *Kluyveromyces lactis* LAC9 transcription factor DNA binding domain (DBD) comprises amino acid residues 85-228 of LAC9 (GenBank Acc. No. AAA35266).

9. A method according to claim 8, wherein the LAC9-DBD comprises amino acid residues 1-228.

10. A method according to claims 1-4, wherein the LAC9-DBD replaces the DNA binding domain of the nuclear receptor.

11. A method according to claims 1-4, wherein the LAC9 response element is the 17-mer consensus sequence 5'-CGGNNNNN(A/T)NNNNNCCG-3' (where "N" represents any nucleotide),

12. A method according to claim 11, wherein said sequence is selected from the LAC9 and GAL4 response elements.

13. A method according to claim 12, wherein said sequence corresponds to the *K. lactis* GAL 1-GAL 10 intergenic region.

14. A method according to claims 2-4, wherein the reporter gene is selected from luciferase, green fluorescent protein, chloramphenicol acyl-transferase.

15. A method according to claim 5, wherein the steroid/thyroid nuclear receptor or intracellular receptor is the human Estrogen receptor alpha.

16. A nucleic acid molecule encoding the *Kluyveromyces lactis* LAC9 transcription factor DNA binding domain (DBD) fused to a nuclear receptor or a portion thereof containing the ligand binding domain.

17. A nucleic acid molecule according to claim 16, wherein the nuclear receptor is a member of the steroid/thyroid hormone receptor family.

18. A nucleic acid molecule according to claim 16, wherein the *Kluyveromyces lactis* LAC9 transcription factor DNA binding domain (DBD) comprises amino acid residues 85-228 of LAC9 (GenBank Acc. No. AAA35266).

19. A nucleic acid molecule according to claim 16, wherein the LAC9-DBD comprises amino acid residues 1-228 of LAC9.

20. A nucleic acid molecule according to claim 17, wherein the nuclear receptor is the human estrogen receptor alpha.

21. A vector containing the nucleic acid molecule of claims 16-20.

22. A eukaryotic or prokaryotic host cell bearing the vector of claim 21.

23. A eukaryotic or prokaryotic host cell according to claim 22, which additionally carries a vector containing a LAC9 response element operatively linked to a gene.

24. The use of a *Kluyveromyces lactis* LAC9 transcription factor DNA binding domain (DBD) fused to a nuclear receptor or a portion thereof containing the ligand binding domain, for the determination of the expression of a target gene functionally linked to a LAC9 response elements in a cultured eukaryotic or prokaryotic cell.
